# EUROPEAN PATENT SPECIFICATION

(11) **EP 1 794 584 B1**
(45) Date of publication and mention of the grant of the patent: **13.06.2018**
(21) Application number: 05796639.2
(22) Date of filing: 15.09.2005
(51) Int. Cl.: G01N 33/28, G01N 25/08

(54) **PORTABLE APPARATUS FOR ANALYSIS OF A REFINERY FEEDSTOCK OR A PRODUCT OF A REFINERY PROCESS**
TRAGBARE VORRICHTUNG ZUR ANALYSE EINES RAFFINERIE-ROHMATERIALS ODER EINES RAFFINERIE-PRODUKTES
DISPOSITIF PORTATIF POUR L'ANALYSE D'UN PRODUIT DE CHARGE D'ALIMENTATION DE RAFFINERIE OU D'UN PRODUIT RESULTANT D'UN PROCEDE DE RAFFINAGE

(30) Priority: 17.09.2004 US 611050 P; 17.09.2004 US 611002 P
(43) Date of publication of application: 13.06.2007
(73) Proprietor: BP OIL INTERNATIONAL LIMITED, Sunbury-on-Thames, Middlesex TW16 7BP (GB)
(72) Inventor: GUDDE, Nicholas, John, Windlesham Surrey GU20 6PL (GB); HODGES, Michael, Wonersh, Surrey GU5 05A (GB); BUTLER, Graham, Churt, Surrey GU10 2HS (GB); VOELKENING, Joachim, Naperville, IL 60564 (US)
(74) Representative: Hamer, Christopher K.
(86) International application number: PCT/US2005/033240
(87) International publication number: WO 2006/034069

(56) References cited:
- EP-A- 0 859 236
- WO-A-00/39561
- US-A1- 2003 037 603
- SEINSCHE K ET AL: "ANWENDUNG DER PLS-REGRESSIONSMETHODE AUF DATEN DER GASCHROMATOGRAPHIE UND DER IR-SPEKTROSKOPIE. QUALITY CONTROL OF JET FUELS - PLS REGRESSION ANALYSIS OF GASCHROMATOGRAPHIC AND IR-SPECTROSCOPIC DATA" ERDOEL ERDGAS KOHLE, URBAN VERLAG, HAMBURG, DE, vol. 112, no. 6, June 1996 (1996-06), pages 261-263, XP000641535 ISSN: 0179-3187
- Ryan L Hartman ET AL: "Distillation in microchemical systems using capillary forces and segmented flow", Lab on a Chip, 7 April 2009 (2009-04-07), pages 1843-1849, XP055123923, DOI: 10.1039/b901790a Retrieved from the Internet: URL:www.rsc.org/loc [retrieved on 2014-06-18]

## Description

### Background of the Invention

This invention relates to a portable apparatus for analysis of a refinery feedstock or of a product of a refinery process.

Feedstock analysis, for example crude oil assay, is an important analysis that must be performed before feedstocks are refined in an oil refinery. Typically, an oil refinery will refine a large number of different feedstocks, including different crude oils, and blends of crude oils, each of which may differ in a number of important properties. In order to ascertain the optimum conditions for refining of each feedstock and to evaluate the potential value of a feedstock, such as the product yields, qualities and values obtainable, and the potential effects on the refining process of said feedstock, such as corrosion or deposition, often a large number of properties need to be analysed. Further impacts on refining processes such as corrosion, fouling or catalyst poisoning are normally not measured in an assay but estimated from other properties.

Traditionally, this has been a time-consuming analysis, requiring a relatively large volume of material and taking 1-2 weeks to produce a partial set of analysis (assay) data, and up to 6 weeks to produce a full set. It is not uncommon for feedstocks to be purchased without a full assay being available to the purchaser, and, hence, the purchaser must make a number of assumptions on the value of the feedstock, for example, for a crude oil this may be based on knowledge of previous crude oils from similar regions. This presents particular risk in the valuation of new crude oil production, particularly from new production areas. It would be advantageous if a refinery feedstock analysis, preferably a full assay, were rapidly available to the potential purchaser.

In addition, rapid analysis of products of refinery processes is also desirable. Such products include intermediates in the overall refinery process, bitumen, products from the overall refinery process which are subsequently used as chemical feedstocks and products from the overall refinery process which are subsequently used as fuels or lubricants, or as blending components for fuels or lubricants, as well as the fuels (e.g. aviation, gasoline, diesel and marine fuels) and lubricants themselves.

Descriptions of refinery processes, and the products therefrom, are well-known to the person skilled in the art, and are described, for example, under the chapter entitled "Oil Refining", by Walther W. Irion and Otto S. Neuwirth, in Ullmann's Encyclopedia of Industrial Chemistry, published by Wiley.

K Seinsche et al., "Anwendung der PLS-Regressionsmethode auf Daten der Gaschromatographie und der IR-Spektroskopie", Erdöl Erdgas Kohle 112 (1996) discloses a fixed apparatus for analysing jet fuel by use of gaschromatographic and IR-spectroscopic data in a two step process.

EP 0 859 236 is directed to a method for controlling the separation of a component in crude oil, in which the oil is analysed at a fixed point by near infra red (NIR) spectroscopy.

WO 00/39561 relates to an automatic analysis method of crude oils using spectroscopy. Although the use of spectroscopy can give rapid analysis of a number of the crude oil properties required for an assay, the method of WO 00/39561 still requires a significant quantity of crude oil, uses conventional distillation equipment and takes 2 days for an analysis, and the infra red spectra used for measurement does not contain information on all the properties normally required for a crude oil assay and thus some properties will be determined by secondary correlations at greatly reduced accuracy.

A method has now been found whereby a determinative assay of a refinery feedstock or a product of a refinery process can be obtained from measurement only of the boiling point profile, density, and total acid number ("TAN") of the feedstock or product, optionally together with a measurement of sulphur content. Previously, it was not appreciated that a useful assay could be obtained using only these parameters. The present invention provides a portable apparatus using which such an assay can be carried out rapidly and away from an analytical laboratory.

Accordingly, the present invention provides a portable apparatus for analysis of a refinery feedstock or a product of a refinery process, said apparatus comprising:
(a) a first microfabricated portable analytical device for determination of the boiling point profile of a refinery feedstock or a product of a refinery process, and
(b) at least two further microfabricated portable analytical devices each of which contains or is associated with a database and an algorithm, at least one of said at least two further devices being adapted for determination of the density of the refinery feedstock or product of a refinery process, and at least one of said at least two further devices being adapted for determination of the total acid number (TAN) of the refinery feedstock or product of a refinery process.

The analytical devices present in the portable apparatus according to the invention are microfabricated, and may be in the form of sensors. Microfabricated devices are devices in which the crucial analytical part or detector of the device is fabricated using techniques consistent with the micro-chip industry, and produces a spectrum or a simple electrical signal, in response to contact with a test substance. A simple electrical signal is fed to an associated set of electronics which either converts the input signal into a value for the property being measured, or further processes the signal using chemometric techniques. A spectrum may be used directly or mathematically treated before being subjected to chemometric techniques to yield the required property or properties. In either case, the value or spectrum is fed to a model generated from the relationship between values or spectra measured and the known composition or properties of such samples determined by previous analytical measurements.

In general, sensors produce a simple electrical signal, are extremely small and cheap, and are used to measure a single property. Other micro devices which produce a spectrum may be somewhat larger and more expensive, and may often be used to measure more than one property.

It is a key feature of the present invention that the apparatus according to the invention should contain at least one device capable of determining the boiling point profile of the refinery feedstock or product of a refinery process, together with at least two other devices chosen to be those devices which are best suited to the property being measured. This matching of measurement device to property being determined differentiates from traditional methods where one device, typically an NIR spectrometer, has been used to measure all required properties via correlations between the spectra and previous analytical measurements.

The first analytical device for determination of the boiling point profile may be capable of yielding the boiling point profile directly, but preferably yields the boiling point profile by using suitable software or models as described above, such as by reference to a suitable database comprising data of known samples, such as of knowo crude oils. By this means, very rapid analysis can be obtained in comparison with traditional methods, which require the oil to be physically separated into its components. The first analytical device preferably determines the true boiling profile (TBP) of a refinery feedstock or a product of a refinery process. The first analytical device is preferably selected from (i) a micro-NIR spectrometer, (ii) a micro-oscillator device and (iii) a micro GC.

The apparatus also comprises at least two further analytical devices, including at least one for measuring density and at least one for measuring TAN. Preferably the device for measuring density is an oscillating sensor, and the device for measuring TAN is an electrochemical sensor. Each of these additional devices may if desired also be capable of determining the boiling point profile, and preferably capable of determining the TBP, in addition to one or more further properties.

Thus, in a preferred embodiment, the present invention provides a portable apparatus for analysis of a refinery feedstock or a product of a refinery process, wherein said apparatus comprises three or more analytical devices selected from a micro-NIR spectrometer, a micro-oscillator device and a micro-GC. In a particularly preferred embodiment, the apparatus contains at least one, preferably at least three, devices selected from a micro-NIR spectrometer, a micro-oscillator device and a micro GC, together with an additional oscillating sensor and an electrochemical sensor.

Typically, the apparatus described above, for example containing three or more of micro-NIR, micro-oscillator and micro-GC devices can provide a significant quantity of the analysis data required for a refinery feedstock assay or analysis of a product of a refinery process.

In addition, the further analytical devices of (b) may comprise a number of additional analytical devices to ascertain further required properties of the refinery feedstock or the product of a refinery process. Further, more than one device may be included in the apparatus to measure a single property. This apparent redundancy may be very valuable as the results can be used to cross-check each other.

Further properties of the refinery feedstock or product of a refinery process which it may be desired to determine in addition to the boiling point profile, density and TAN will be sample dependent, and typically may include total base number (TBN), cold flow properties (such as pour point, freezing point and cloud point), viscosity, Research Octane Number (RON), Motor Octane Number (MON), cetane number, smoke point, Bureau of Mines Correlation Index (BMCI), refractive index, conductivity, sulphur content, nitrogen content, nickel content, vanadium content and combinations thereof. Preferably the apparatus contains at least one additional device for determining one or more of these properties. For example, the apparatus may contain an additional device for determining sulphur content, for example a pyrolyser coupled with a micro GC and a micro mass spectrometer, and/or an additional device for determining metal content, e.g. a metal specific sensor.

Suitable devices for determination of said further properties may include micro conductivity/capacitance devices (e.g. for acidity), micro rheological devices (e.g. for viscosity) and micro spectroscopic devices, such as NIR, ion mobility/differential mobility, acousto-optical, acoustic, UV-Vis and Mid-IR spectroscopies (e.g. MID IR for naphthenic acidity). Micro-conductivity/capacitance devices, micro rheological devices, and acoustooptical devices are all available in the form of sensors and can form part of an array of sensors in the apparatus of the invention.

The one or more further analytical devices in step (b) may determine density and TAN and optionally one or more additional properties of the refinery feedstock or product of a refinery process as a whole, and/or density and TAN and optionally one or more additional properties of one or more fractions of the refinery feedstock or product of a refinery process.

Thus, in a further preferred embodiment, the present invention provides a portable apparatus for analysis of a refinery feedstock or a product of a refinery process, said apparatus comprising:
(a) a micro-separation device, capable of determination of the boiling point profile of a refinery feedstock or a product of a refinery process, for separation of the refinery feedstock or product of a refinery process into two or more fractions, and
(b) two or more further analytical devices for determination of density and TAN of one or more of the fractions.

In this embodiment, the micro-separation device provides separation of the refinery feedstock or product of a refinery process into at least two fractions, and said fractions can then be analysed by two or more further analytical devices.

The micro-separation device of step (a) is capable of determination of the boiling point profile, and preferably is capable of determination of the TBP, of a refinery feedstock or a product of a refinery process. Preferably, the micro-separation device is the first analytical device of step (a) of the present invention. Most preferably, the micro-separation device is a micro-oscillator device.

Alternatively, the two or more further analytical devices of (b) may comprise said first analytical device (a) for determination of the boiling point profile, preferably of the TBP, in addition to two or more analytical devices for the determination of density and TAN of the fractions.

The further analytical devices of (b) are used for determination of density, TAN and optionally boiling point profile of one or more of the fractions produced in step (a). One or more further analytical devices may also be provided which can determine properties of the refinery feedstock or product of a refinery process as a whole.

Alternatively, or in addition, the further analytical devices may also comprise a number of other analytical devices, as described above, to ascertain further required properties of the total refinery feedstock or product of a refinery process and/or of the fractions.

Preferably the apparatus according to the present invention is hand-held, suitably having a total weight of less than 5 kg, such as 2kg or less.

The apparatus according to the present invention requires only a small quantity of refinery feedstock or product of a refinery process (hereinafter the refinery feedstock or product of a refinery process may be referred to as "sample"), typically less than 100ml, such as 10 ml or less, and preferably 1 ml or less. Because of the small quantity of sample required the analysis can be performed in a significantly shorter time than conventional analysis, such as conventional crude oil assay.

Typically, the apparatus according to the present invention provides an analysis in less than two hours, and preferably provides an analysis in less than 30 minutes, preferably less than 5 minutes, such as less than 2 minutes.

The refinery feedstock may be any suitable feedstock that may be fed to a refinery, such as a crude oil, a synthetic crude (syncrude), a biocomponent, an intermediate, such as a residue or a cracked stock, or blends of one or more of said feedstocks.

Preferably the refinery feedstock is a crude oil or blend of crude oils, optionally also comprising (blended with) one or more of a synthetic crude component, a biocomponent or an intermediate component, such as a residue component or a cracked stock component.

Where the portable apparatus of the present invention is used for analysis of a product of a refinery process, the product may be an intermediate stream in the overall refinery process, a bitumen, a product from the overall refinery process which is subsequently used as a chemical feedstock, a product from the overall refinery process which is subsequently used as a fuel or lubricant, or as a blending component for a fuel or lubricant, or a fuel, for example an aviation, gasoline, diesel or marine fuel or lubricant itself.

The micro-oscillator device, when present, is preferably an acoustic optical device or sensor. Micro-oscillator devices are based on measurement of the frequency of oscillation of the device, which changes with mass of material on the oscillator. Thus, if material evaporates or condenses on the device, the frequency changes. As well as information on boiling point profile, acoustic optical devices may provide information on viscosity, cold flow properties, volatile contaminants and deposits formation. Suitable micro-oscillators are described in U.S. Patent Nos. 5,661,233 and 5,827,952.

Micro-NIR, when present, may be used, for example, to provide information on boiling point profile and to give a simulated distillation curve, as well as to provide information on density and amounts of saturates and aromatics in the sample as a whole and/or in fractions obtained from a suitable separation step. Sulphur and/or cold flow properties, such as cloud point and freezing point, acidity (TAN), Research Octane Number (RON), Motor Octane Number (MON), cetane number and smoke point may also be measured. Suitable micro-NIR analysers include the Axsun NIR-APS Analyser produced by Axsun Technologies Inc., Massachusetts.

Micro-GC, when present, may provide a simulated distillation curve and can provide hydrocarbon speciation, such as of e1-e9 hydrocarbons. Suitable micro-Ge devices include SLS Micro-technology Ges or other micro-chip based Ges such as those being developed by the University of Massachusetts.

Micro-ion mobility/differential mobility spectrometry, when present, may be used to provide information on specific molecular types and particularly on polar molecules in the sample, for example contaminants such as organic chlorides or methanol, as well as sulphides and nitrogen compounds. Further, micro-ion mobility/differential mobility spectrometry coupled with a micro pyrolyser, can give enhanced nitrogen and sulphur analysis. Micro-ion mobility/differential mobility spectrometry is best implemented in combination with micro GC and/or a pre-fractionation/preconcentration device. Suitable micro- ion mobility/differential mobility spectrometers include the Sionex microDMx.

Micro-fabricated devices in the form of sensors are advantageous because of their small size and low cost. In a preferred embodiment of the apparatus according to the invention, the apparatus contains one or more micro devices which are sensors. A plurality of such sensors may be present, preferably arranged in the form of an array. Each sensor will be provided with an associated electronics to convert the sensor signal to a value and optionally a chemometric model to relate the value to the desired property. The sensor signal will contain information on the property being measured, or which is directly related to the property being measured.

The present invention has the advantage that, due to their relatively small individual sizes and sample requirements, a number of different analytical devices may be arranged in a single portable apparatus. The apparatus according to the present invention includes at least 3 different analytical devices, preferably at least 5 different analytical devices, such as at least 10 different analytical devices, allowing a number of properties of a sample (or of fractions thereof) to be ascertained using the apparatus, and providing a significant amount of data for the analysis, either directly or via a suitable database model as described further below.

Due to its portability, the apparatus according to the present invention can be taken to the location of the sample to be analysed, and a rapid analysis of the sample obtained. For example, for crude oil analysis (assay), the apparatus may be used for "at location" rapid assessment/valuation of crude oils, for example on a crude oil tanker or in a land-based crude oil storage tank, during the loading or discharge of a crude oil tanker in a port or from a pipeline, or at an oil exploration drilling or production site, allowing the value of the crude oil to a potential purchaser to be quickly ascertained. At an oil exploration drilling site, the apparatus of the present invention may be used at the "well-head" on the drilling site to provide rapid analysis of a crude oil, for example, to provide rapid feedback of the properties of a crude oil at a test well allowing evaluation of said crude oil. In such application the oil may be passed through a pre-filter to remove contamination from the drilling process, for example from drilling additives, or suitable correction models may be used to correct the data obtained.

Because of the relatively small size of the components of the apparatus according to the present invention, the power requirements are also relatively low. Hence, the apparatus may be operated from a suitable battery (or battery pack), preferably a rechargeable battery, without the battery requirements being too heavy to impact the portability of the apparatus.

Preferably the apparatus comprises, or is at least compatible with, wireless communications, such as a wireless mesh network, and more preferably, with remote communications means, such as satellite-based data communication, such that the analysis results may be readily communicated to the potential purchaser, again reducing the timescale on which the analysis data is available to the potential purchaser.

Especially where suitable micro-devices are not available, the apparatus according to the present invention may be used in combination with other portable analysers, particularly those yielding elemental data, such as portable X-Ray Fluorescence (XRF) spectroscopy and Laser Induced Breakdown Spectroscopy (LIBS) to improve the breadth of assay.

XRF, for example, can provide analysis of sulphur and metals content of a sample, for example of crude oil fractions. Suitable, portable, XRF analysers include those available from OXFORD instruments.

Generally, the apparatus according to the present invention, optionally in combination with any other analysers, will generate data in respect of at least 1 O key properties of the sample to be analysed, such as at least 20 key properties. For crude oil assay, for example, the apparatus according to the present invention, optionally in combination with any other analysers, preferably provides data in respect of, or from which can be derived (as described further below), the majority of the key properties measured in a conventional crude oil assay, which include the boiling point profile, density, total acid number (TAN), cold flow properties (such as pour point and cloud point), viscosity, sulphur content, nitrogen content, nickel content, vanadium content and combinations thereof of the full crude oil and/or of fractions thereof. Similar properties are required for assay of other refinery feedstocks.

The analysis data from the apparatus according to the present invention, and optionally any other analysers, may directly generate sufficient analysis data, for example assay data for the crude oil being analysed or valued.

Alternatively, the analysis data obtained directly from the apparatus, optionally in combination with other analysers, may be enhanced via input to a suitable database model, typically a model derived from analysis data obtained from analysis of a large number of other samples. For example, for analysis of a crude oil, the analysis data obtained may be enhanced via input to a crude oil assay database model derived from assay data obtained from analysis of a large number of other crude oils. The crude oil assay database model may be used to generate a detailed assay with improved confidence.

Similarly, for analysis a product from a refinery process, the data may be enhanced via input to a suitable database model of the product properties derived from analysis of a large number of similar (equivalent) products.

As an example, where the analysis data obtained from the apparatus according to the present invention, and optionally any other analysers, is data from a multivariate analytical technique, such as NIR, the analysis data may be analysed by fitting the information to a linear combination of known multivariate analytical data in said database, such as described in WO 03/48759.

Because of the rapid analysis obtainable from the apparatus of the present invention, analyses can be obtained more often and/or can be used for process optimisation. For example, the apparatus may be used at a refinery and regular analyses can be performed on blends of refinery feedstocks, such as blends of crude oils, produced (from two or more sources available) at the refinery, to ensure optimum configuration of the refinery for the blend. Further the apparatus may be used to verify consistency and/or quality of feedstocks on arrival at a refinery or blending station and/or may be used to provide on-line or at-line determination of feedstock quality and property data for input to blending and process refinery optimisation models.

Where the apparatus of the present invention is used at the "well-head" on a drilling site, a number of apparatus' may be operated at different well-heads which use a common transport mechanism, for example a common pipeline, to provide analysis of the crude oil from each well. Analysis of the individual crude oils and appropriate scheduling may allow more optimum composition of the final crude oil blend. In addition, by repeated analysis of the crude oils from different well-heads, changes in the individual crude oils with time can be used to predict the effects on the produced crude oil blend, or influence the blending to maintain a constant quality crude oil blend.

Similarly, where the apparatus is used for analysis of a product obtainable from a refinery process, the apparatus may be used to check consistency and quality of the product at the refinery, or at subsequent locations, such as at chemical plants themselves, at fuels blending terminals or in fuel-containing tanks, such as in fuel tankers or stationary tanks at airports, dockyards or on petrol station forecourts.

The present disclosure also provides a method for analysis of a refinery feedstock or a product of a refinery process, said method comprising analysing the refinery feedstock or product of a refinery process using the portable apparatus previously described.

The method may also comprise analysis of the refinery feedstock or product of a refinery process with one or more further portable analysers, communication of the analysis results to a potential purchaser, and/or combination of the analysis information obtained with a database model as previously described.

## Claims

1. A portable apparatus for analysis of a refinery feedstock or a product of a refinery process, said apparatus comprising:
(a) a first microfabricated portable analytical device for determination of the boiling point profile of a refinery feedstock or a product of a refinery process, and
(b) at least two further microfabricated portable analytical devices each of which contains or is associated with a database and an algorithm, at least one of said at least two further devices being adapted for determination of the density of the refinery feedstock or product of a refinery process, and at least one of said at least two further devices being adapted for determination of the total acid number of the refinery feedstock or product of a refinery process.

2. A portable apparatus as claimed in claim 1, wherein the first microfabricated analytical device is selected from (i) a micro-NIR spectrometer, (ii) a micro-oscillator device and (iii) a micro GC.

3. A portable apparatus as claimed in either claim 1 or claim 2, wherein at least one of the at least two further microfabricated analytical devices comprises an analytical device that is capable of determining the boiling point profile in addition to one or more further properties.

4. A portable apparatus as claimed in any one of the preceding claims, wherein the first microfabricated portable analytical device and the at least two further microfabricated analytical devices comprise three or more analytical devices selected from a micro-NIR spectrometer, a micro-oscillator device and a micro-GC.

5. A portable apparatus as claimed in any one of the preceding claims in which at least one of the first and at least two further analytical devices is a sensor.

6. A portable apparatus as claimed in claim 5, wherein the first microfabricated portable analytical device and the at least two further microfabricated analytical devices comprise three or more analytical devices selected from a micro-NIR spectrometer, a micro-oscillator device and a micro-GC, together with an oscillating sensor and an electrochemical sensor.

7. A portable apparatus as claimed in any one of the preceding claims, which comprises at least one device for determining sulphur content, and/or at least one device for determining metal content.

8. A portable apparatus as claimed in any one of the preceding claims, which is additionally capable of measuring at least one of the following properties: total base number, any cold flow property, viscosity, Research Octane Number, Motor Octane Number, cetane number, smoke point, Bureau of Mines Correlation Index, refractive index, conductivity, nitrogen content, and combinations thereof.

9. A portable apparatus as claimed in any one of the preceding claims, wherein the at least two further microfabricated portable analytical devices in step (b) are adapted to determine density and TAN of the refinery feedstock or product of a refinery process as a whole and/or density and TAN of one or more fractions of the refinery feedstock or product of a refinery process.

## Patentansprüche

1. Tragbare Vorrichtung zur Analyse eines Raffinerie-Rohmaterials oder eines Raffinerie-Produktes, umfassend:
(a) eine erste mikrofabrizierte tragbare analytische Vorrichtung zur Bestimmung des Siedepunktprofils eines Raffinerie-Rohmaterials oder eines Raffinerieproduktes, und
(b) mindestens zwei weitere mikrofabrizierte tragbare analytische Vorrichtungen, die jeweils eine Datenbank und einen Algorithmus enthalten bzw. einer/einem solchen zugeordnet sind, wobei mindestens eine dieser zwei weiteren Vorrichtungen auf die Bestimmung der Dichte des Raffinerie-Rohmaterials oder des Raffinerie-Produktes eingerichtet ist, und mindestens eine dieser zwei weiteren Vorrichtungen auf die Bestimmung der Gesamtsäurezahl des Raffinerie-Rohmaterials oder des Raffinerie-Produktes eingerichtet ist.

2. Tragbare Vorrichtung nach Anspruch 1, wobei die erste mikrofabrizierte analytische Vorrichtung aus der Gruppe (i) Mikro-NIR Spektrometer, (ii) Mikro-Oszillatorgerät oder (iii) Mikro-GC ausgewählt wird.

3. Tragbare Vorrichtung nach entweder Anspruch 1 oder Anspruch 2, wobei mindestens eine der mindestens zwei weiteren mikrofabrizierten analytischen Vorrichtungen eine analytische Vorrichtung ist, die fähig ist, neben dem Siedepunktprofil eine oder mehrere weitere Eigenschaften zu bestimmen.

4. Tragbare Vorrichtung nach einem der vorhergehenden Ansprüche, wobei die erste mikrofabrizierte analytischen Vorrichtung und die mindestens zwei weiteren mikrofabrizierten analytischen Vorrichtungen drei oder mehr analytische Vorrichtungen sind, die aus der Gruppe Mikro-NIR Spektrometer, Mikro-Oszillatorgerät oder Mikro-GC ausgewählt werden.

5. Tragbare Vorrichtung nach einem der vorhergehenden Ansprüche, wobei mindestens die erste und/oder mindestens zwei weitere analytische Vorrichtungen Sensoren sind.

6. Tragbare Vorrichtung nach Anspruch 5, wobei die erste mikrofabrizierte analytischen Vorrichtung und die mindestens zwei weiteren mikrofabrizierten analytischen Vorrichtungen drei oder mehr analytische Vorrichtungen sind, die aus der Gruppe Mikro-NIR Spektrometer, Mikro-Oszillatorgerät und Mikro-GC, zusammen mit einem oszillierenden Sensor und einem elektrochemischen Sensor ausgewählt werden.

7. Tragbare Vorrichtung nach einem der vorhergehenden Ansprüche, die mindestens eine Vorrichtung zur Bestimmung des Schwefelgehaltes und/oder mindestens eine Vorrichtung zur Bestimmung des Metallgehaltes aufweist.

8. Tragbare Vorrichtung nach einem der vorhergehenden Ansprüche, die außerdem fähig ist, mindestens eine der folgenden Eigenschaften zu messen: Gesamtbasenzahl, Kaltfließverhalten jeglicher Art, Viskosität, Research Oktanzahl, Motoroktanzahl, Cetanzahl, Rauchpunkt, Korrelationsindex des Bureau of Mines, Brechungsindex, Leitfähigkeit, Stickstoffgehalt, und Kombinationen derselben.

9. Tragbare Vorrichtung nach einem der vorhergehenden Ansprüche, wobei die mindestens zwei weiteren mikrofabrizierten tragbaren analytischen Vorrichtungen in Schritt (b) auf die Bestimmung von Dichte und TAN des Raffinerie-Rohmaterials oder des Raffinerie-Produktes als Ganzes und/oder auf die Bestimmung von Dichte und TAN einer oder mehrerer Fraktionen des Raffinerie-Rohmaterials oder des Raffinerie-Produktes eingerichtet sind.

## Revendications

1. Appareil portatif pour l'analyse d'une charge de raffinage ou d'un produit d'un processus de raffinage, ledit appareil comprenant :
(a) un premier dispositif d'analyse portatif microfabriqué pour la détermination d'un profil de point d'ébullition d'une charge de raffinage ou d'un produit d'un processus de raffinage, et
(b) au moins deux dispositifs d'analyse portatifs microfabriqués supplémentaires, dont chacun contient ou est associé à une base de données et un algorithme, au moins un desdits au moins deux dispositifs supplémentaires étant conçus pour la détermination de la densité de la charge de raffinage ou du produit d'un processus de raffinage, et au moins un desdits au moins deux dispositifs supplémentaires étant conçus pour la détermination de l'indice d'acide total de la charge de raffinage ou du produit d'un processus de raffinage.

2. Appareil portatif selon la revendication 1, dans lequel le premier dispositif d'analyse microfabriqué est sélectionné parmi (i) un micro-spectromètre par proche infrarouge (NIR), (ii) un dispositif micro-oscillateur et (iii) un micro-chromatographe en phase gazeuse (GC).

3. Appareil portatif selon la revendication 1 ou la revendication 2, dans lequel au moins un des au moins deux dispositifs d'analyse microfabriqués supplémentaires comprend un dispositif d'analyse qui est capable de déterminer le profil de point d'ébullition en plus d'une ou de plusieurs propriétés supplémentaires.

4. Appareil portatif selon l'une quelconque des revendications précédentes, dans lequel le premier dispositif portatif d'analyse microfabriqué et les au moins deux dispositifs d'analyse microfabriqués supplémentaires comprennent trois dispositifs d'analyse ou plus sélectionnés parmi un micro-spectromètre par proche infrarouge (NIR), un dispositif micro-oscillateur et un micro-chromatographe en phase gazeuse (GC).

5. Appareil portatif selon l'une quelconque des revendications précédentes dans lequel au moins un dispositif parmi le premier dispositif d'analyse et les au moins deux dispositifs d'analyse est un capteur.

6. Appareil portatif selon la revendication 5, dans lequel le premier dispositif d'analyse portatif microfabriqué et les au moins deux dispositifs d'analyse microfabriqués supplémentaires comprennent trois dispositifs d'analyse ou plus sélectionnés parmi un micro-spectromètre par proche infrarouge (NIR), un dispositif micro-oscillateur et un micro-chromatographe en phase gazeuse (GC), conjointement avec une sonde oscillante et un capteur électrochimique.

7. Appareil portatif selon l'une quelconque des revendications précédentes, qui comprend au moins un dispositif pour déterminer la teneur en soufre, et/ou au moins un dispositif pour déterminer la teneur en métal.

8. Appareil portatif selon l'une quelconque des revendications précédentes, qui est en plus capable de mesurer au moins une des propriétés suivantes : l'indice d'alcalinité totale, toute propriété d'écoulement à froid, la viscosité, l'indice d'octane recherche, l'indice d'octane moteur, l'indice de cétane, le point de fumée, l'indice de corrélation du Bureau of Mines, l'indice de réfraction, la conductivité, la teneur en azote, et des combinaisons de ceux-ci.

9. Appareil portatif selon l'une quelconque des revendications précédentes, dans lequel les au moins deux dispositifs d'analyse portatifs microfabriqués supplémentaires dans l'étape (b) sont conçus pour déterminer la densité et l'indice d'acide total de la charge de raffinage ou du produit d'un processus de raffinage dans l'ensemble et/ou la densité et l'indice d'acide total d'une ou de plusieurs fractions de la charge de raffinage ou du produit d'un processus de raffinage.
